(19) Europäisches Patentamt
European Patent Office
Office européen des brevets

(11) Veröffentlichungsnummer: **0 388 698 A1**

(12) # EUROPÄISCHE PATENTANMELDUNG

(21) Anmeldenummer: **90104180.6**

(51) Int. Cl.⁵: **C07D 251/34, B29C 33/60, //B29K69:00**

(22) Anmeldetag: **03.03.90**

(30) Priorität: **16.03.89 DE 3908598**

(43) Veröffentlichungstag der Anmeldung:
**26.09.90 Patentblatt 90/39**

(84) Benannte Vertragsstaaten:
**DE FR GB IT**

(71) Anmelder: **BAYER AG**

**D-5090 Leverkusen 1 Bayerwerk(DE)**

(72) Erfinder: **Ohst, Holger, Dr.**
**Klutstein 33**
**D-5060 Bergisch Gladbach 2(DE)**
Erfinder: **Meier, Helmut-Martin, Dr.**
**Am Obersthof 3**
**D-4030 Ratingen 6(DE)**
Erfinder: **Kircher, Klaus, Dr.**
**Alfred-Kubin-Strasse 3**
**D-5090 Leverkusen 1(DE)**

(54) **Formtrennmittel für Poly(ester)carbonate.**

(57) Gegenstand der vorliegenden Erfindung sind nach speziellen Verfahren hergestellte trimerisierte aliphatische Isocyanate, ihre Verwendung als Formtrennmittel in thermoplastischen, aromatischen Polycarbonaten und/oder thermoplastischen, aromatischen Polyestercarbonaten und/oder thermoplastischen, aromatischen Polyestern, gegebenenfalls unter Mitverwendung von üblichen Additiven, die so erhaltenen leicht entformbaren Formmassen auf Basis der thermoplastischen Polycarbonate, Polyestercarbonate und/oder Polyester sowie Verfahren zur Herstellung dieser leicht entformbaren Formmassen.

EP 0 388 698 A1

## Formtrennmittel für Poly(ester)carbonate

Es ist bekannt, daß verschiedene Katalysatoren für die Trimerisierung von Alkylisocyanaten eingesetzt werden können. So sind z.B. beschrieben Lithiumoxid, Natriummethoxid, Natriumformiat, Natriumcarbonat, Natriumbenzoat, Natriumborhydrid, Kalium-tert.-butylat, Alkaliseifen, Bleisalze, Titantetrabutylat, Triethylamin, Oxalsäure, organische Verbindungen des vierwertigen Zinns sowie Triethylphosphan als Katalysatoren zuzusetzen (H. Ulrich, Cycloaddition Reactions of Heterocumulenes, 1967, S. 128 ff., Academic Press)

Trialkylphosphane und Arylalkylphosphane sowie metallorganische Verbindungen von Elementen der chemischen Gruppen IVa, IVb, Va und IIb des PSE sind ebenfalls als Katalysatoren beschrieben (S. Patai, The Chemistry of Cyanates and Thio-Derivatives, 1977, S. 674 ff).

Auch sind Trialkylphosphane als Katalysatoren genannt bei Kirk-Othmer, Encyclopedia of Chemical Technology, Vol. 13, S. 794, 1981.

Langkettige aliphatische Isocyanurate sind bekannt und wurden verwendet als Elastomerzusatz (EP 136.898), als Entschäumungsmittel (DOS 1 965 643), als wasserabweisendes Mittel (C.A.-Nr. 55, 24564 c) und als Mittel für Insektizidwirkung (C.A.-Nr. 104, 16499 n). Weiterhin sind Trialkylisocyanurate genannt als Fließverbesserer in hochmolekularen aromatischen Polycarbonaten und Polyestercarbonaten (US 4.243.570).

Als Entformungsmittel für Polycarbonat ist eine Vielzahl von Verbindungen beschrieben worden, z.B. Fettsäuren (EP 189.572), Ketone (EP 100.918), Vinylether (US 4.438.234), N,N-Diallylamide (4.554.302), Siloxane (US 3.751.519), Paraffine und Alkane (US 4.415.696, US 4.626.565), Perfluoralkylsulfonamide (DOS 2.506.726) und insbesondere Fettsäureester (EP 103.107, DOS 2.729.485, US 4.131.575, US 4.097.435, US 3.836.499).

In dem Maße, in dem jede Verbindungsklasse in den vorstehend genannten Patentschriften für die Verwendung als Entformungsmittel für Polycarbonat sachdienlich ist, wird hier besonders auf diese verwiesen. Jede der genannten Verbindungsklassen in Polycarbonat erfüllt in einzelnen Punkten neben guter Wirkung als Entformungsmittel die jeweiligen zusätzlichen Anforderungen, als da sind Transparenz, geringe Verfärbung und geringer Molekulargewichtsabbau bei thermischer Belastung. Sofern in allen genannten Punkten diese zusätzlichen Anforderungen gleichzeitig zu erfüllen sind, erweisen sich die oben angeführten Verbindungsklassen als Entformungsmittel als ungenügend.

Es zeigt sich, daß alle genannten Stoffklassen bei Berücksichtigung aller Anforderungen an einen modernen Polycarbonat-Formstoff Nachteile aufweisen, sei es die Beeinflussung der Transparenz (z.B. Siloxane, Paraffine und Alkane) oder sei es der Molekulargewichtsabbau des Polycarbonats/Polyestercarbonats; insbesondere die Fettsäureester beeinflussen durch Umesterung und damit Abbau bei hohen Temperaturen das thermische Langzeitverhalten negativ. In der Praxis besteht trotz der Vielzahl der in Patentschriften beschriebenen Entformungsmittel noch ein Bedarf an einer Komponente, die keinen negativen Einfluß auf Farbe und Transparenz und keinen signifikanten Einfluß auf mechanische, elektrische und thermische Kurzzeitwerte hat, und die bei sehr gutem Verhalten als Entformungsmittel keinen signifikanten Einfluß auf das thermische Langzeitverhalten ausübt.

Überraschenderweise erweist sich ein mit Triarylphosphan als Katalysator aus entsprechenden Isocyanaten hergestelltes langkettiges aliphatisches Isocyanurat als ein gut wirksames Entformungsmittel in Polycarbonat und/oder aromatischen Polyestercarbonaten und/oder aromatischen Polyestern, ohne die obengenannten Nachteile zu zeigen.

Nach dem Stand der Technik hergestellte aliphatische Isocyanurate erweisen sich bei Einsatz in Polycarbonat als problematisch. Sowohl saure (z.B. organische Zinnverbindungen) als auch basische Katalysatoren (z.B. Natriummethoxid, Triethylphosphan) führen in Polycarbonat zu starkem Molekulargewichtsabbau. Eine quantitative Entfernung dieser Katalysatoren aus dem gebildeten Iso cyanurat ist mit technisch vertretbarem Aufwand nicht möglich (Destillation, Kristallisation oder Neutralisation). Eine Trimerisierung ohne Katalysator gelingt nicht.

Überraschenderweise ist eine Trimerisierung von aliphatischen Isocyanaten möglich mit Triarylphosphanen, insbesondere wirkt Triphenylphosphan geeignet als Katalysator.

Gegenstand der vorliegenden Erfindung ist somit ein Verfahren zur Trimerisierung von aliphatischen Isocyanaten bei Temperaturen von 50° bis 250°, vorzugsweise von 150° bis 220° C und bei Drucken von 0,1 bar bis 100 bar, vorzugsweise von 1 bar bis 10 bar entweder an Luft oder unter Schutzgasatmosphäre in Anwesenheit von Katalysatoren, das dadurch gekennzeichnet ist, daß man Triarylphosphane in Mengen von 0,1 Gew.-% bis 10 Gew.-%, vorzugsweise in Mengen von 1 Gew.-% bis 5 Gew.-%, bezogen auf das eingesetzte aliphatische Isocyanat, einsetzt und die Reaktion bis zu einer NCO-Zahl von 0 % durchführt.

(Bezüglich NCO-Zahl siehe Vieweg-Höchtlen, Kunststoff-Handbuch, Bd. VII, 1966, Seite 90).

Als Schutzgas dient beispielsweise $N_2$.

Gegenstand der vorliegenden Erfindung sind außerdem die nach dem erfindungsgemäßen Verfahren erhältlichen Isocyanurate.

Gegenstand der vorliegenden Erfindung ist außerdem die Verwendung der erfindungsgemäß erhältlichen Isocyanurate als Entformungsmittel für thermoplastische, aromatische Polycarbonate, für thermoplastische, aromatische Polyestercarbonate und für thermoplastische, aromatische Polyester.

Gegenstand der vorliegenden Erfindung sind außerdem Mischungen von thermoplastischen, aromatischen Polycarbonaten und/oder von thermoplastischen, aromatischen Polyestercarbonaten und/oder von thermoplastischen, aromatischen Polyestern mit den erfindungsgemäß erhältlichen Isocyanuraten in Mengen von 0,05 Gew.-% bis 3 Gew.-%, vorzugsweise von 0,1 Gew.-% bis 1 Gew.-%, bezogen auf das Gewicht des Polycarbonats und/oder des Polyestercarbonats und/oder des Polyesters.

Wie bereits erwähnt, wird der Katalysator, also das Triarylphosphan nicht aus dem trimerisierten Isocyanurat entfernt, so daß er bei der Verwendung des Isocyanurats als Entformungsmittel in die Polycarbonate und in die Polyestercarbonate und in die Polyester eingearbeitet wird. Das Triarylphosphan kann aber auch in größeren Mengen, bezogen auf Entformungsmittelgemisch, eingesetzt werden, und zwar bis etwa maximal 65 Gew.-%, bezogen auf Gewichtssumme Isocyanurat + Triarylphosphan.

Gegenstand der vorliegenden Erfindung sind somit auch Gemische aus trimerisiertem aliphatischen Isocyanat und Triarylphosphan, wobei das trimerisierte aliphatische Isocyanat in Mengen von 99,9 Gew.-% bis 35 Gew.-% und das Triarylphosphan in Mengen von 0,1 Gew.-% bis 65 Gew.-%, bezogen auf die Gewichtssumme aus Isocyanurat und Triarylphosphan, eingesetzt werden.

Gegenstand der vorliegenden Erfindung ist außerdem die Verwendung des erfindungsgemäßen Gemisches aus trimerisiertem, aliphatischem Isocyanat und Triarylphosphan als Entformungsmittel für Polycarbonate und für Polyestercarbonate und für Polyester und für Mischungen aus Polycarbonaten und/oder Polyestercarbonaten und/oder Polyestern.

Bei Einsatz dieses Gemisches liegt die Gewichtssumme an Isocyanurat und Triarylphosphan im Polycarbonat beziehungsweise im Polyestercarbonat, beziehungsweise im Polyester, wieder wie vorstehend erwähnt, von 0,05 bis 3, vorzugsweise von 0,1 bis 1 Gew.-%.

Gegenstand der vorliegenden Erfindung sind außerdem Mischungen von thermoplastischen, aromatischen Polycarbonaten und/oder von thermoplastischen, aromatischen Polyestercarbonaten und/oder von thermoplastischen, aromatischen Polyestern mit den trimerisierten aliphatischen Isocyanaten und mit den Triarylphosphanen, wobei der Gehalt an erfindungsgemäßem Gemisch aus trimerisiertem, aliphatischem Isocyanat und aus Triarylphosphan wiederum von 0,05 Gew.-% bis 3 Gew.-%, vorzugsweise von 0,1 Gew.-% bis 1 Gew.-%, bezogen jeweils auf das Gewicht des Polycarbonats und/oder des Polyestercarbonats und/oder des Polyesters, liegt.

Wie bereits erwähnt, ist aus dem US-Patent 4 243 570 bekannt, Isocyanurate, beispielsweise auch Trialkylisocyanurate als Fließverbesserer für Polycarbonate und Polyestercarbonate zu verwenden. Die Herstellung der Isocyanurate ist hierbei nicht spezifiziert, vielmehr wird ausgeführt, daß die Isocyanurate nach den bekannten Methoden des Standes der Technik hergestellt werden können, beispielsweise nach "Kirk-Othmer's Encyclopedia of Chemical Technology, Vol. 20, Seiten 662 bis 671, John Wiley and Sons, 1969, New York (Spalte 2, Zeilen 3 bis 8 von US-Patent 4 243 570).

Aus der EP-PS 0 143 906 und der dort zitierten Literatur ist Triarylphosphan als Stabilisator für Polycarbonat bekannt.

Die Kombination beider Lit.-Stellen legt unseres Erachtens die Verwendung von Gemischen aus aliphatischen Isocyanuraten mit Triarylphosphanen als Entformungsmittel nicht nahe, zumal Entformung und Fließverbesserung Vorgänge sind, die nur in Ausnahmefällen identisch sind.

Es ist bekannt, die Fließfähigkeit von Polycarbonat durch Zugabe geringer Anteile an Polyalkylenterephthalaten und/oder organischen Phosphorsäureestern zu verbessern; die mit diesen Anteilen erreichbare Fließfähigkeitsverbesserung ist jedoch mit keiner signifikanten Verbesserung des Entformungsverhaltens verknüpft. Andererseits wirken die als gute Entformungshilfsmittel bekannten Ester langkettiger organischer Säuren und Alkohole nicht signifikant als Fließverbesserer; eine Fließverbesserung wird nur dann mit Hilfe üblicher Entformungsmittel erreicht, wenn alkalische oder aminische Verunreinigungen beim Verarbeitungsvorgang das Polymermolekül schädigen und zu einer Molekulargewichtsverkleinerung führen, was in der Praxis unerwünscht ist.

Geeignete aliphatische Isocyanate sind sowohl aliphatische im engeren Sinn, also solche mit Alkyl-Resten, aber auch cycloaliphatische Isocyanate, also solche mit Cycloalkyl-Resten.

Als Isocyanate sind sowohl Mono-Isocyanate als auch Di-Isocyanate zu verstehen. Als gemeinsamer Begriff für die hier in Rede stehenden Isocyanate seien deshalb (cyclo)aliphatische (Mono- und Di-)Isocyanate gewählt.

Bevorzugte (cyclo)aliphatische Mono-Isocyanate sind solche der Formel (1)

$R\text{-}N=C=O$ (1),

worin R ein $C_1$-$C_{30}$-Alkyl-Rest oder ein $C_4$-$C_{12}$-Cycloalkyl-Rest ist, wobei $C_{12}$-$C_{24}$-Alkyl-Reste besonders bevorzugt sind.

Bevorzugte (cyclo)aliphatische Di-Isocyanate sind solche der Formel (2)

$OCN\text{-}R'\text{-}NCO$ (2),

worin $R'$ ein $C_2$-$C_{14}$-Alkylen oder ein $C_4$-$C_{12}$-Cycloalkylen ist, wobei $C_6$-$C_{10}$-Alkylene und $C_6$-$C_{10}$-Cycloalkylene besonders bevorzugt sind.

Die (cyclo)aliphatischen Di-Isocyanate werden nur zusammen mit den (cyclo)aliphatischen Mono-Isocyanaten eingesetzt und zwar in Mengen bis zu 10 Gew.-%, vorzugsweise bis zu 2 Gew.-%, bezogen auf das Gewicht des jeweils eingesetzten Mono-Isocyanats.

Erfindungsgemäß einzusetzende Triarylphosphane sind solche mit Arylresten aus 6 bis 14 C-Atomen, die gegebenenfalls ein- oder mehrfach substituiert sind, und zwar durch Alkyl-, Aryl- oder Halogensubstituenten.

Bevorzugte Triarylphosphane sind die der Formel (3)

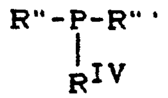

(3),

worin $R''$, $R'''$ und $R^{IV}$ Phenyl- oder Naphthyl bedeuten, die noch durch $CH_3$, $C_2H_5$, $C_6H_5$, F, Cl oder Br ein- oder mehrfach substituiert sein können.

Als Mono-Isocyanate seien z.B. genannt:

Ethylisocyanat, Propylisocyanat, Butylisocyanat, Isopropylisocyanat, sec.-Butylisocyanat, Isobutylisocyanat, tert.-Butylisocyanat, Dodecylisocyanat, Hexadecylisocyanat, Stearylisocyanat, Cyclohexylisocyanat, 3,3,5-Trimethylcyclohexylisocyanat, 2-Norbornylmethylisocyanat.

Bevorzugte Mono-Isocyanate sind z.B. Dodecylisocyanat, Hexadecylisocyanat, Stearylisocyanat sowie Gemische dieser Isocyanate.

Als Di-Isocyanate seien z.B. genannt: Hexamethylendiisocyanat, 2,2,4-Trimethyl-1,6-diisocyanatohexan, 1,4-Diisocyanatocyclohexan, 3,3,5-Trimethyl-1-isocyanato-3-isocyanatomethylcyclohexan, Dimeryldiisocyanat, Bis(4-isocyanato-cyclohexyl)methan.

Bevorzugtes Di-Isocyanat ist z.B. Hexamethylendiisocyanat.

Als Triarylphosphane sind beispielsweise genannt: Triphenylphosphan, Tris-p-tolylphosphan, Tris-(p-nonylphenyl)phosphan, Trisnaphthylphosphan, Tris-(p-chlorphenyl)phosphan, Tris-(p-fluorphenyl)phosphan und Mesityl-phenyl-o-chlorphenyl-phosphan.

Besonders bevorzugt ist Triphenylphosphan.

Sowohl die Isocyanate als auch die Triarylphosphane können allein als auch im Gemisch zweier verschiedener Isocyanate bzw. zweier verschiedener Triarylphosphane eingesetzt werden, mit dem Vorbehalt, daß die Summe der jeweils eingesetzten Di-Isocyanate maximal 10 Gew.-% der Summe der jeweils eingesetzten Mono-Isocyanate beträgt.

Die resultierenden trimerisierten Isocyanate haben vorzugsweise die Strukturformel (4a)

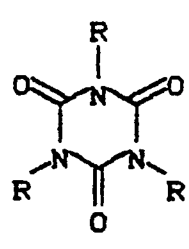

(4a)

oder die Strukturformel (4b)

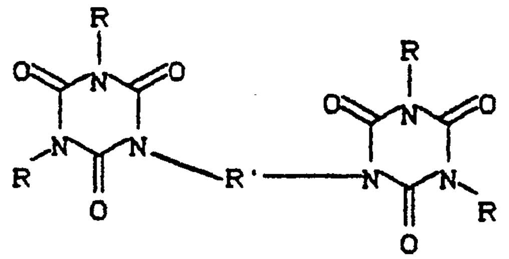 (4b) oder

oligomere Strukturen mit mehr als zwei Isocyanurat-Ringen also solche mit mindestens einer Struktureinheit (4c)

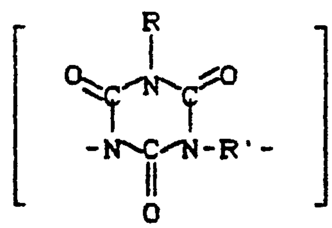 (4c) und im

Falle von Verzweigungen mit mindestens einer Struktureinheit (4d)

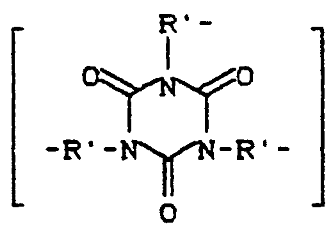 (4d),

worin jeweils R und R′ die für die Formeln (1) und (2) genannte Bedeutung haben. Gemische der Strukturen (4a) mit (4b) und/oder mit solchen auf Basis (4c) und/oder (4d) können gegebenenfalls vorliegen.

Die nach dem erfindungsgemäßen Verfahren erhältlichen trimerisierten Isocyanate fallen als glasartig erstarrte Schmelzen an, die pulverisierbar sind.

Sofern die erfindungsgemäßen Gemische aus den trimerisierten aliphatischen Isocyanaten und den Triarylphosphanen anders als durch die erfindungsgemäße Trimerisierung der aliphatischen Isocyanate in Gegenwart von Triarylphosphanen hergestellt werden sollen, geht man zweckmäßigerweise beispielsweise wie folgt vor:

Durch Alkylierung von Isocyanursäure sind i.a. dreifach substituierte Isocyanurate erhältlich. So ist z.B. die Alkylierung von Isocyanursäure mit n-Hexylchlorid bei erhöhten Temperaturen in einer Ausbeute von 71 % in Kirk-Othmer, Encyclopedia of Chemical Technology, Vol. 7, S. 401, 1979 beschrieben. Diese dreifach alkylierten Isocyanuarate können dann in den gewünschten Mengen mit den Triarylphosphanen zu den erfindungsgemäßen Entformungsmitteln in bekannten Weise vermischt werden.

Thermoplastische aromatische Polycarbonate im Sinne der vorliegenden Erfindung sind die durch Umsetzung von Diphenolen, insbesondere von Dihydroxydiarylalkanen, mit Phosgen oder Diestern der Kohlensäure erhältlichen Polykondensate, wobei außer den unsubstituierten Dihydroxydiarylalkanen auch solche geeignet sind, deren Arylreste in o- und/oder m-Stellung zur Hydroxylgruppe Methylgruppen oder Halogenatome tragen. Ebenso sind verzweigte Polycarbonate geeignet. Als Kettenabbrecher dienen beispielsweise Monophenole. Als Verzweiger beispielsweise Trisphenole oder Tetraphenole.

Die Polycarbonate haben mittlere Gewichtsmittelmolekulargewichte $\overline{M}_w$ zwischen 10 000 und 300 000, vorzugsweise zwischen 50 000 und 250 000 einseitig und 20 000 bis 40 000 andererseits, ermittelt durch Gelpermeationschromatographie oder durch Messungen der rel. Viskosität in $CH_2Cl_2$ bei 25 °C und einer

Konzentration von 0,5 g/pro 100 ml.

Geeignete Diphenole sind z.B. Hydrochinon, Resorcin, 4,4′-Dihydroxydiphenyl, Bis-(hydroxy-phenyl)-alkane wie beispielsweise $C_1$-$C_8$-Alkylen- bzw. $C_2$-$C_8$-Alkylidenbisphenole, Bis-(hydroxyphenyl)-cycloalkane wie beispielsweise gegebenenfalls substituierte $C_5$-$C_{15}$-Cycloalkylen-bzw. gegebenenfalls substituierte $C_5$-$C_{15}$-Cycloalkylidenbisphenole, Bis-(hydroxy-phenyl)-sulfide, -ether, -ketone, -sulfoxide oder -sulfone. Ferner $\alpha,\alpha'$ -Bis-(hydroxyphenyl)-diisopropylbenzol sowie die entsprechenden kernalkylierten bzw kernhalogenierten Verbindungen.

Bevorzugt sind Polycarbonate auf Basis Bis-(4-hydroxyphenyl)-propan-.2,2 (Bisphenol A), bis-(4-hydroxy-3,5-dichlor-phenyl)-propan-2,2 (Tetrachlorbisphenol A), Bis-(4-hdyroxy-3,5-dibromphenyl)-propan-2,2 (Tetrabrombisphenol A), Bis-(4-hydroxy-3,5-dimethyl-phenyl)-propan-2,2 (Tetramethylbisphenol A), Bis-(4-hydroxy-phenyl)-cyclohexan-1,1 (Bisphenol Z), 1,1-Bis-(4-hydroxyphenyl)-3,3,5-trimethylcyclohexan (TMC-Bisphenol) gemäß deutscher Patentanmeldung P 3 832 396.6 (Le A 26 344) sowie auf Basis von Dreikernbisphenolen wie $\alpha,\alpha'$-Bis-(4-hydroxyphenyl)-p-diisopropylbenzol. Weitere geeignete Diphenole sowie Herstellung der Polycarbonate sind beispielsweise in den US-Patenten 3 028 365, 3 062 781 und 3 879 347 beschrieben.

Verzweigte Polycarbonte sind beispielsweise im US-Patent 4 185 009 bzw. im deutschen Patent 2 500 092 beschrieben.

Thermoplastische, aromatische Polyester im Sinne vorliegender Erfindung sind solche auf Basis von Diphenolen, aromatischen Dicarbonsäuredichloriden, Kettenabbrechern und gegebenenfalls Verzweigungsmitteln.

Als Diphenole dienen die für die Polycarbonatherstellung vorstehend genannten Verbindungen.

Als Kettenabbrecher dienen Monophenole, als Verzweiger dienen Trisphenole und Tetraphenole.

Darüberhinaus können als Verzweigungsmittel hier bevorzugt auch aromatische Tricarbonsäuretrichloride oder aromatische Tetracarbonsäuretetrachloride oder Säurechloride von noch höherwertigeren aromatischen Carbonsäuren eingesetzt werden.

Sie werden in Mengen von 0,01 bis 1 Mol-%, bezogen auf eingesetzte aromatische Dicarbonsäuredichloride, eingesetzt, während bei Einsatz von phenolischen Verzweigern deren Menge von 0,01 bis 1 Mol-% sich auf eingesetzte Diphenole zur Herstellung des aromatischen Polyesters bezieht.

Verzweigungsmittel zur Herstellung aromatischer Polyester sind beispielsweise in der DE-OS 2 940 024, Seiten 9/10 (Le A 19 932) beschrieben.

Geeignete aromatische Dicarbonsäuredichloride sind:
Terephthalsäuredichlorid,
Isophthalsäuredichlorid,
o-Phthalsäuredichlorid,
Diphenyl-dicarbonsäure-dichlorid,
Diphenylether-dicarbonsäure-dichlorid,
Naphthalindicarbonsäure-dichlorid und deren Gemische.

Bevorzugte Gemische sind solche von Terephthalsäuredichloriden mit Isophthalsäuredichloriden im Verhältnis 20:1 bis 1:20, insbesondere von 7:3 bis 3:7.

Die Herstellung der aromatischen Polyester aus Säurechloriden, Diphenolen, Kettenabbrechern und gegebenenfalls Verzweigern erfolgt vorzugsweise nach dem Ver fahren der Phasengrenzflächenpolykondensation in bekannter Weise. (Siehe dazu beispielsweise wiederum die DE-OS 2 940 024 und für das TMC-Bisphenol deutsche Patentanmeldung P 3 903 103.9 (Le A 26 313)).

Thermoplastische aromatische Polyestercarbonate im Sinne vorliegender Erfindung sind solche erhältlich in bekannter Weise aus Diphenolen, Phosgen, aromatischen Dicarbonsäuredichloriden, Kettenabbrechern und gegebenenfalls Verzweigern. Polyestercarbonate sowie ihre Herstellung sind bekannt (siehe beispielsweise EP-OS 0 036 080 (Le A 20 203) und US-PS 3 169 121 und für das TMC-Bisphenol deutsche Patentanmeldung P 3 903 103.9 (Le A 26 313)).

Geeignete Diphenole sind die für die Polycarbonatherstellung bereits genannten.

Geeignete aromatische Dicarbonsäuredichloride sind die für aromatische Polyesterherstellung bereits genannten, wobei wiederum Gemische von Terephthalsäuredichloriden mit Isophthalsäuredichloriden in den bereits erwähnten Mischungsverhältnissen besonders geeignet sind.

Geeignete Kettenabbrecher sind Monophenole, wie sie auch für die Polycarbonat- und Polyester-Herstellung geeignet sind.

Geeignete Verzweiger sind die vorstehend für die aromatischen Polyester genannten mehr als zweiwertigen Phenole und mehr als zweiwertigen aromatischen Carbonsäurechloride.

Die aromatischen Polyestercarbonate im Sinne der vorliegenden Erfindung haben bis etwa 80 Mol-%, vorzugsweise bis etwa 50 Mol-% Carbonatgruppen, bezogen auf die Molsumme an Carbonatgruppen und

aromatischen Carbonsäureestergruppen.

Sowohl der Ester- als auch der Carbonatanteil der erfindungsgemäßen aromatischen Polyestercarbonate kann in Form von Blöcken oder statistisch verteilt im Polykondensat vorliegen.

Die relative Lösungsviskosität ($\eta$rel) der aromatischen Polyester und Polyestercarbonate liegt im Bereich 1,18 bis 1,4 vorzugsweise 1,22 bis 1,3 (gemessen an Lösungen von 0,5 g Polyester beziehungsweise Polyestercarbonat in 100 ml $CH_2Cl_2$-Lösung bei 25 °C).

Die Einarbeitung der erfindungsgemäß erhältlichen, trimerisierten Isocyanate bzw. der erfindungsgemäßen Gemische aus den separat hergestellten Isocyanuraten und den Triarylphosphanen in die thermoplastischen aromatischen Polycarbonate und/oder die thermoplastischen aromatischen Polyestercarbonate und/oder die thermoplastischen, aromatischen Polyester kann beispielsweise durch Auftrudeln auf das Kunststoffgranulat, anschließende Homogenisierung im Extruder und erneute Granulierung erfolgen; die Einarbeitung kann aber auch schon während der Herstellung der Polycarbonate und/oder der Polyestercarbonate und/oder der Polyester erfolgen.

Vor oder während oder nach der Einarbeitung der erfindungsgemäßen Entformungsmittel können weitere für Polycarbonate, Polyestercarbonate oder für Polyester übliche Additive in den üblichen Mengen den erfindungsgemäßen Formmassen in bekannter Weise eingearbeitet werden, beispielsweise Schlagzähmodifikatoren, Stabilisatoren, Flammschutzmittel, Pigmente sowie Füllstoffe und Verstärkungsstoffe.

Gegenstand der vorliegenden Erfindungen sind außerdem Mischungen von thermoplastischen, aromatischen Polycarbonaten und/oder von thermoplastischen aromatischen Polyestercarbonaten und/oder von thermoplastischen, aromatischen Polyestern mit den erfindungsgemäß zu verwendenden Formtrennmitteln sowie mit mindestens einem üblichen Zusatz ausgewählt aus Schlagzähmodifikatoren, Stabilisatoren, Flammschutzmitteln, Pigmenten, Füllstoffen und Verstärkungsstoffen in den üblichen Mengen.

Gegenstand der vorliegenden Erfindung ist somit auch ein Verfahren zur Herstellung von thermoplastischen Formmassen aus thermoplastischen aromatischen Polycarbonaten und/oder thermoplastischen, aromatischen Polyestercarbonaten und/oder thermoplastischen, aromatischen Polyestern, das dadurch gekennzeichnet ist, daß man die erfindungsgemäß erhältlichen Entformungsmittel, also die erfindungsgemäß trimerisierten Isocyanate oder die erfindungsgemäßen Gemische aus Isocyanuraten und Triarylphosphanen, in Mengen von 0,05 Gew.-% bis 3 Gew.-%, vorzugsweise in Mengen von 0,1 Gew.-% bis 1 Gew.-%, bezogen auf thermoplastisches Polycarbonat und/oder thermoplastisches Polyestercarbonat und/oder thermoplastischem Polyester, diesen Thermoplasten in üblicher Weise, während oder nach der Herstellung dieser Thermoplasten zumischt, die erhaltene Mischung anschließend bei Temperaturen von 220 °C bis 420 °C homogenisiert und danach granuliert, wobei gegebenenfalls vor, während oder nach der Einarbeitung der erfindungsgemäßen Formtrennmittel übliche Additive, ausgewählt aus Schlagzähmodifikatoren, Stabilisatoren, Flammschutzmitteln, Pigmenten, Füllstoffen und Verstärkungsstoffen den thermoplastischen Polycarbonaten und/oder den thermoplastischen Polyestercarbonaten und/oder den thermoplastischen Polyestern in den üblichen Mengen in bekannter Weise eingearbeitet werden.

Die erfindungsgemäße Einarbeitung der erfindungsgemäßen Formtrennmittel und gegebenenfalls der genannten üblichen Additive kann auch dadurch variiert werden, daß die Thermoplasten nach erfolgter Zumischung nicht erneut granuliert werden, sondern sogleich zu den gewünschten Formkörpern bzw. zu anderem Halbzeug als Granulat bzw. zu Fertigartikeln verarbeitet werden.

Wie bereits angesprochen, können die erfindungsgemäßen Formtrennmittel und gegebenenfalls die genannten üblichen Additive den thermoplastischen Polycarbonaten und/oder den thermoplastischen Polyestercarbonaten und/oder den thermoplastischen Polyestern auch während der Herstellung dieser Thermoplaste, beispielsweise den nach der Herstellung dieser Thermoplaste anfallenden organischen Lösungen vor deren Eindampfung zugesetzt werden. Die anschließende Eindampfung und Verarbeitung zu Granulat oder Formkörpern kann dann in üblicher Weise erfolgen.

Beispiele für übliche Additive sind Phosphitstabilisatoren, Epoxide gegen Hydrolyse, Antioxidantien, Mittel für die Lösungsmittelbeständigkeit, Stabilisatoren gegen UV-Einwirkung, Tropf-Inhibitoren zwecks Verbesserung der Brandwidrigkeit, inerte Füllstoffe und aktive Füllstoffe wie auch aktive Pigmente, wobei in deren Aktivität vor der Einarbeitung in bekannter Weise modifiziert werden kann.

Die erfindungsgemäßen thermoplastischen Mischungen bzw. Formmassen können in bekannter Weise auf Spritzgießmaschinen zu Formkörpern oder in Extrudern zu Halbzeug oder Folien verarbeitet werden.

Der Zusatz der erfindungsgemäß zu verwendenden Entformungsmittel macht sich bei aromatischen Polycarbonaten oder Polyestercarbonaten weder in der Transparenz noch in der Farbe noch im Molekulargewichtsabbau bei thermischer Belastung nachteilig bemerkbar. Auch bei aromatischen Polyestern sind keine negativen Einflüsse erkennbar.

Durch die erfindungsgemäße Verwendung der erfindungsgemäßen Formtrennmittel werden der Entformungsdruck beim Spritzguß erniedrigt und fehlerfreie Artikel mit guter Oberflächenbeschaffenheit erhalten.

Gegenstand der vorliegenden Erfindung ist somit auch die Verarbeitung der erfindungsgemäßen Formmassen nach dem Spritzgußverfahren.

Die erfindungsgemäßen Formmassen finden als Formkörper bzw. Spritzgußartikel überall dort Verwendung, wo die vorhandenen Werkzeuge eine Entformungsmittel-haltige Formmasse erfordern und hohe Ansprüche an die thermische Stabilität über lange Zeit an die Formteile gestellt werden.

In den nachfolgenden Beispielen sind alle Prozentsätze der Zusätze als Gewichtsprozente des thermoplastischen Harzes angegeben.

Vergleichsbeispiel 1 (ohne Katalysator)

100 g Stearylisocyanat wurden unter Stickstoff bei 200 °C gerührt. Nach 13 Stunden konnte keine Abnahme der Isocyanat-Zahl beobachtet werden. NCO-Zahl: 14,4 %.

Beispiel 1

100 g Stearylisocyanat und 5 g Triphenylphosphan wurden 35 Stunden bei 200 °C unter Stickstoff gerührt. NCO-Zahl: 0 %.

Vergleichsbeispiel 2 (mit üblichem Katalysator)

100 g Stearylisocyanat und 2 g Dibutylzinnoxid wurden unter Stickstoff bei 200 °C bis zur NCO-Zahl von 0 % zur Reaktion gebracht.

Beispiel 2

Allgemeine Herstellungsvorschrift für das verwendete Polycarbonat

Ca. 454 Teile 4,4'-Dihydroxydiphenyl-2,2-propan und 9,5 Teile p-tert.-Butylphenol werden in 1,5 l Wasser suspendiert. In einem 3-Halskolben, ausgestattet mit Rührer und Gaseinleitungsrohr, wird der Sauerstoff aus der Reaktionsmischung entfernt, indem unter Rühren 15 min. lang Stickstoff durch die Reaktionsmischung geleitet wird. Dann werden 355 Teile 45 %iger Natronlauge und 1000 Teile Methylenchlorid zugegeben. Die Mischung wird auf 25 °C abgekühlt. Unter Aufrechterhaltung dieser Temperatur durch Kühlen werden 237 Teile Phosgen während einer Zeitdauer von 120 min. zugegeben. Eine zusätzliche Menge von 75 Teilen einer 45 %igen Natronlauge wird nach 15 bis 30 Minuten zugegeben bzw. nachdem die Phosgenaufnahme begonnen hat. Zu der entstandenen Lösung werden 1,6 Teile Triethylamin zugegeben und die Mischung weitere 15 min. gerührt. Eine hochviskose Lösung wird erhalten, deren Viskosität durch Zugabe von Methylenchlorid reguliert wird. Die wäßrige Phase wird abgetrennt. Die organische Phase wird mit Wasser salz- und alkalifrei gewaschen. Das Polycarbonat wird aus der gewaschenen Lösung isoliert und getrocknet. Das Polycarbonat hat eine relative Viskosität von 1,29 bis 1,30, gemessen an einer 0,5 %igen Lösung in Methylenchlorid bei 20 °C. Das entspricht ungefähr einem Molekulargewicht von 32 000. Das so gewonnene Polycarbonat wird extrudiert und granuliert.

Beispiel 3

Auf das gemäß Beispiel 2 hergestellte Polycarbonat-Granulat wird in einer Trommel bei Raumtemperatur die in Tabelle 1 angegebene Menge Entformungsmittel aufgetrudelt, anschließend über einen Extruder bei 280 °C zu einem Strang extrudiert und granuliert.

Tabelle 1

| Beispiel | Teile Polycarbonat gemäß Beispiel 2 | Teile Entformungsmittel |
|---|---|---|
| 3a **) | 100 | 0 |
| 3b **) | 99,5 | 0,5 (Pentaerythrittetrastearat) *) |
| 3c **) | 99,5 | 0,5 (gemäß Vergleichsbeispiel 2) |
| 3d | 99,5 | 0,5 (gemäß Beispiel 1) |

*) Bei dem verwendeten Fettsäureester handelt es sich um Loxiol VPG 861®, ein Handelsprodukt der Fa. Henkel.
**) Die Beispiele 3a, 3b und 3c dienen als Vergleichsbeispiele.

Entformungswirkung

Die Wirksamkeit der erfindungsgemäß zu verwendenden Entformungsmittel wird anhand der bei der Entformung von Spritzgußmassen benötigten Entformungskräfte gemessen. Diese werden in diesem Beispiel dadurch gemessen, daß man den in dem Ölzylinder der Auswerferhydraulik bei der Entformung sich aufbauenden Druck über ein elektronisches Aufzeichnungssystem über den gesamten Entformungs vorgang des Spritzgußteiles mit der Geometrie eines Zylinders von 35 mm Länge und einem Durchmesser von 40 mm mit einer Wandstärke von 2 mm zeitlich verfolgt. In der Tabelle 2 ist die im zeitlichen Mittel benötigte Entformungskraft aufgeführt für die Beispiele 3a bis d und für unterschiedliche Werkzeugtemperaturen:

Tabelle 2

| Beispiel | Werkzeugtemperatur | zeitlich gemittelte Entformungskraft *) |
|---|---|---|
| 3a | 90 °C | 140 bar |
| | 110 °C | 82 bar |
| | 130 °C | 50 bar |
| 3b | 90 °C | 93 bar |
| | 110 °C | 48 bar |
| | 130 °C | 12 bar |
| 3c | 90 °C | 49 bar |
| | 110 °C | 24 bar |
| | 130 °C | 10 bar |
| 3d | 90 °C | 47 bar |
| | 110 °C | 24 bar |
| | 130 °C | 8 bar |

*) Integration einer Entformungskraft-Zeit-Kurve dividiert durch die Zeitdauer des Entformungsvorgangs.

Alterungsverhalten

Das Alterungsverhalten bei thermischer Belastung wurde anhand des Molekulargewichtsabbaus überprüft. Dazu wurden Granulate nach Einarbeitung der erfindungsgemäß zu verwendenden Entformungsmittel bzw. nach Regranulierung (Beispiel 3a) des aromatischen Polycarbonats bei 155 °C in einem Umluftofen gelagert und der Molekulargewichtsabbau anhand der relativen Lösungsviskosität (5 g Granulat gelöst in 1 l

Methylenchlorid, gemessen bei 25° C, DIN 51.562 Pl.3) verfolgt.

Tabelle 3

| | Beispiel | | | |
|---|---|---|---|---|
| | 3a | 3b | 3c | 3d |
| Ausgangswert | 1,298 | 1,296 | 1,280 | 1,293 |
| 500 Stunden-Wert | 1,292 | 1,233 | 1,184 | 1,283 |
| 1000 Stunden-Wert | 1,285 | 1,208 | - | 1,258 |

Transparenz und Farbe

Bei einer Verarbeitungstemperatur von 300° C hergestellte Spritzgußkörper wurden bezüglich Transparenz und Farbe anhand der Transmission und des Yelloxness-Index vermessen. Die Resultate sind Tabelle 4 zu entnehmen.

Tabelle 4

| Beispiel | Transmission * | Yellowness-Index * |
|---|---|---|
| 3a | 87,00 % | 5,9 |
| 3b | 88,37 % | 4,2 |
| 3c | 84,57 % | 16,3 |
| 3d | 87,34 % | 6,2 |

*) gemessen an 4 mm dicken Prüfplatten bei monochromatischer Probenbeleuchtung mit der Meßgeometrie O/d an Diano-Spektralphotometer gemäß DIN 5033.

**Ansprüche**

1. Verfahren zur Trimerisierung von aliphatischen Isocyanaten bei Temperaturen von 50° bis 250° und bei Drucken von 0,1 bis 100 bar entweder an Luft oder unter Schutzgasatmosphäre in Anwesenheit von Katalysatoren, dadurch gekennzeichnet, daß man Triarylphosphane in Mengen von 0,1 bis 10 Gew.-% bezogen auf das eingesetzte aliphatische Isocyanat, einsetzt und die Reaktion bis zu einer NCO-Zahl von 0 % durchführt.

2. Isocyanurate, erhältlich nach dem Verfahren des Anspruchs 1.

3. Verwendung der Isocyanurate des Anspruchs 1 als Entformungsmittel für thermoplastische aromatische Polycarbonate, für thermoplastische aromatische Polyestercarbonate und für thermoplastische aromatische Polyester.

4. Mischungen von thermoplastischen, aromatischen Polycarbonaten und/oder von thermoplastischen, aromatischen Polyestercarbonaten und/oder von thermoplastischen, aromatischen Polyestern mit den erfindungsgemäß erhältlichen Isocyanuraten in Mengen von0,05 bis 3 Gew.-%, bezogen auf das Gewicht des Polycarbonats und/oder des Polyestercarbonats und/oder des Polyesters.

5. Gemische aus trimerisiertem aliphatischen Isocyanat und Triarylphosphan, wobei das trimerisierte aliphatische Isocyanat, in Mengen von 99,9 bis 35 Gew.-% und das Triarylphosphan in Mengen von 0,1 bis 65 Gew.-%, bezogen auf die Gewichtssumme aus Isocyanurat und Trialkylphosphan.

6. Verwendung des Gemisches des Anspruchs 5 als Entformungsmittel für Polycarbonat und für Polyestercarbonate und für Polyester und für Mischungen aus Polycarbonaten und/oder Polyestercarbonaten und/oder Polyestern.

7. Mischungen von thermoplastischen, aromatischen Polycarbonaten und/oder von thermoplastischen, aromatischen Polyestercarbonaten und/oder von thermoplastischen, aromatischen Polyestern mit den trimerisierten aliphatischen Isocyanaten und mit den Triarylphosphanen des Anspruchs 5, wobei der Gehalt an Gemisch des Anspruchs 5, von 0,05 Gew.-% bis 3 Gew.-%, vorzugsweise von 0,1 Gew.-% bis 1 Gew.-% liegt, bezogen jeweils auf das Gewicht des Polycarbonats und/oder des Polyestercarbonats und/oder des Polyesters.

8. Mischungen gemäß Ansprüche 4 oder 7, dadurch gekennzeichnet, daß sie weitere, übliche Additive ausgewählt aus Schlagzähmodifikatoren, Stabilisatoren, Flammschutzmitteln, Pigmenten, Füllstoffen und Verstärkungsstoffen in den üblichen Mengen enthalten.

9. Verfahren zur Herstellung von thermoplastischen Formmassen aus thermoplastischen aromatischen Polycarbonaten und/oder thermoplastischen, aromatischen Polyestercarbonaten und/oder thermoplastischen, aromatischen Polyestern, das dadurch gekennzeichnet ist, daß man die gemäß Anspruch 2 erhältlichen, trimerisierten Isocyanate oder die Gemische aus Isocyanuraten und Triarylphosphanen des Anspruchs 5 in Mengen von 0,05 Gew.-% bis 3 Gew.-%, bezogen auf thermoplastisches Polycarbonat und/oder thermoplastisches Polyestercarbonat und/oder thermoplastischem Polyester, diesen Thermoplasten in üblicher Weise während oder nach der Herstellung dieser Thermoplasten zumischt, die erhaltene Mischung anschließend bei Temperaturen von 220 °C bis 420 °C homogenisiert und danach granuliert, wobei gegebenenfalls vor, während oder nach der Einarbeitung der erfindungsgemäßen Formtrennmittel übliche Additive, ausgewählt aus Schlagzähmodifikatoren, Stabilisatoren, Flammschutzmitteln, Pigmenten, Füllstoffen und Verstärkungsstoffen den thermoplastischen Polycarbonaten und/oder den thermoplastischen Polyestercarbonaten und/oder den thermoplastischen Polyestern in den üblichen Mengen in bekannter Weise eingearbeitet werden.

10. Verarbeitung der Mischungen der Ansprüche 4, 7 oder 8 nach dem Spritzgußverfahren.

Europäisches Patentamt

# EUROPÄISCHER RECHERCHENBERICHT

Nummer der Anmeldung

EP 90 10 4180

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | GB-A-1 166 316 (FARBENFABRIKEN BAYER AG)<br>* Seite 2; Seite 3, Zeilen 55-63; Beispiel 5 *<br>----- | 1 | C 07 D 251/34<br>B 29 C 33/60 //<br>B 29 K 69:00 |
| | | | **RECHERCHIERTE SACHGEBIETE (Int. Cl.5)** |
| | | | C 07 D 251/00 |

Der vorliegende Recherchenbericht wurde für alle Patentansprüche erstellt

| Recherchenort | Abschlußdatum der Recherche | Prüfer |
|---|---|---|
| DEN HAAG | 12-07-1990 | VAN BIJLEN H. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)